# EUROPÄISCHE PATENTSCHRIFT

(11) **EP 0 519 218 B1**
(45) Veröffentlichungstag und Bekanntmachung des Hinweises auf die Patenterteilung: **18.11.1998**
(21) Anmeldenummer: 92108254.1
(22) Anmeldetag: 15.05.1992
(51) Int. Cl.: C12N 15/31, C12N 15/74, C12P 21/02, C07K 14/00, C12N 1/21

(54) **Hybrid-Plasmid für 38 kDa Antigen von M. Tuberculosis, E. coli als Wirt, 38 kDa Antigen und etwa 33 kDa Protein**
Hybrid-plasmid for the 38 kDa antigen from M. tuberculosis, E. coli as host, the 38 kDa antigen and a 33 kDa protein
Plasmide hybride pour l'antigène 38 kDa de M. tuberculosis, E. coli comme hôte, l'antigène de 38 kDa et une protéine d'environ 33 kDa

(30) Priorität: 17.05.1991 DE 4116249
(43) Veröffentlichungstag der Anmeldung: 23.12.1992
(73) Patentinhaber: Gesellschaft für Biotechnologische Forschung mbH (GBF), D-38124 Braunschweig (DE)
(72) Erfinder: Singh, Mahavir, Dr., W-3300 Braunschweig (DE); Timmis, Kenneth, Prof.Dr., W-3300 Braunschweig (DE)
(74) Vertreter: Boeters, Hans Dietrich, Dr.

(56) Entgegenhaltungen:
- INFECTION AND IMMUNITY Bd. 57, Nr. 8, August 1989, WASHINGTON US Seiten 2481 - 2488 ANDERSEN, A. & HANSEN, E. 'Structure and mapping of antigenic domains of protein antigen b, a 38,000-molecular-weight protein of Mycobacterium tuberculosis'
- GENE Bd. 52, 1987, AMSTERDAM NL Seiten 279 - 283 SCHAUDER, B. ET AL. 'Inducible expression vectors incorporating the Escherichia coli atpE translational initiation region'

## Beschreibung

Tuberkulose ist eine höchst ansteckende Erkrankung des Menschen mit über 3 Millionen Todesfällen und 8 Millionen Neuerkrankungen pro Jahr. Das Erscheinen von AIDS wird wahrscheinlich die Situation wegen der Reaktivierung von ruhenden *M*. *tuberculosis* in immunkompromittierten Patienten noch verschlimmern. Die Infektionsdosis bei Tuberkulose ist herausragend niedrig, das heißt, einer bis drei Tuberkelbazillen reichen aus, um in der Lunge einen Primäraffekt zu initiieren. Die Diagnostizierung erkrankter Personen spielt eine wesentliche Rolle bei Epidemiologie, Verhütung und Ausbreitung der Erkrankung. Gegenwärtig basiert die Diagnose auf der Kultivierung von *M. tuberculosis* aus dem Sputum, was wegen des langsamen Wachstums des Organismus etwa 6 Wochen dauert. Ein weiterer wichtiger Teil der Diagnose ist der "Tuberkulin-Test". Tuberkulin oder das gereinigte Proteinderivat davon (PPD) ist eine Mischung von Proteinen aus dem Kulturmediumfiltrat von durch Hitze abgetöteten *M. tuberculosis.* Dieser Test weist aufgrund von Kreuzreaktivitäten in Individuen, die sich mit anderen Mycobakterien infiziert haben oder dagegen geimpft wurden, eine hohe Unspezifität auf. Es bestand also eine dringliche Notwendigkeit, definierte und spezifische serodiagnostische und zur Hauttestung auf Tuberkulose geeignete Reagentien zu entwickeln.
Serologische Studien haben gezeigt, daß das 38 kDa-Antigen von *M. tuberculosis* immundominante Epitope umfaßt, welche für die virulenten Stämme von *M. tuberculosis* spezifisch sind. Dieses Antigen wird in kleinen Quantitäten in dem Impfstamm BCG produziert, der ein avirulentes Derivat des bovinen Tuberkelbazillus *M. bovis* darstellt. So kann, basierend auf serologischen Methoden, dieses Antigen verwendet werden, um zwischen Organismen des *M*. *tuberculosis*-Komplexes und anderen Mycobakterien zu unterscheiden. Die Reinigung des nativen 38 kDa-Antigens direkt aus *M. tuberculosis* ist aufgrund der geringen Ausbeuten, des langsamen Wachstums und der virulenten Natur des Organismus nicht praktikabel.

Wie bereits erwähnt ist *Mycobacterium tuberculosis* der kausale Erreger der Tuberkulose, einer weitverbreiteten Erkrankung des Menschen, die jährlich etwa 3 Millionen Todesopfer fordert. Bedeutende Ziele der Mycobakterien-forschung sind die Bereitstellung einer schützenden Immunität gegen Tuberkulose durch effektivere Impfstoffe und die Entwicklung spezifischer Hauttest/Serodiagnostik-Reagentien. Eine Vorbedingung solcher Ziele ist die Charakterisierung und eingehende Bestimmung der immunologischen Rolle der einzelnen mycobakteriellen Antigene. Zu diesem Zweck werden ausreichende Mengen dieser Antigene benötigt. Die Reinigung von Antigenen direkt aus *M. tuberculosis* ist wegen der geringen Zellausbeuten, des langsamen Waschstums und der virulenten Natur des Organismus schwierig (10, 26). Eine mögliche Lösung dieses Problems ist die Herstellung rekombinanter Antigene in biotechnologisch zugänglichen Organismen wie zum Beispiel *E. coli.*

Die immunologische und diagnostische Relevanz des 38 kDa-Antigenproteins aus *M. tuberculosis* wurde schon gezeigt (2, 26). Das Protein umfaßt speziesspezifische B-Zell-Epitope (2), und T-Zellen, welche aus immunisierten Mäusen, Meerschweinchen oder Menschen isoliert wurden, proliferieren, wenn sie in Gegenwart des Antigens kultiviert werden (10, 24, 26). Die Mehrzahl der Menschen (insbesondere diejenigen des HLA-Typs DR2), welche an aktiver Tuberkulose leiden, entwikkeln Antikörper gegen das 38 kDa-Antigen (4).

Das 38 kDa-Protein des grampositiven Bakteriums *Mycobacterium tuberculosis* H37Rv ist ein immundominantes Antigen von möglicher Nutzbarkeit für die Diagnostik und Entwicklung eines Impfstoffes. Die Abschätzung dieses Potentials erfordert große Mengen des gereinigten Proteins, was schwierig wenn nicht unmöglich sein würde, müßte man es aus *M. tuberculosis* selbst gewinnen.

Aus Infection and Immunology, 57 (1989) 2481 - 2488 ist ein 38 kDa großes Protein von *Myccobacterium tuberculosis* bekannt. Es handelt sich jedoch um kein rekombinantes Produkt, vielmehr um ein natives Protein, das glycosyliert und acetyliert ist. Die Expression eines weder glycosylierten noch acylierten Proteins in einem Mikroorganismus wird nicht vorgeschlagen.

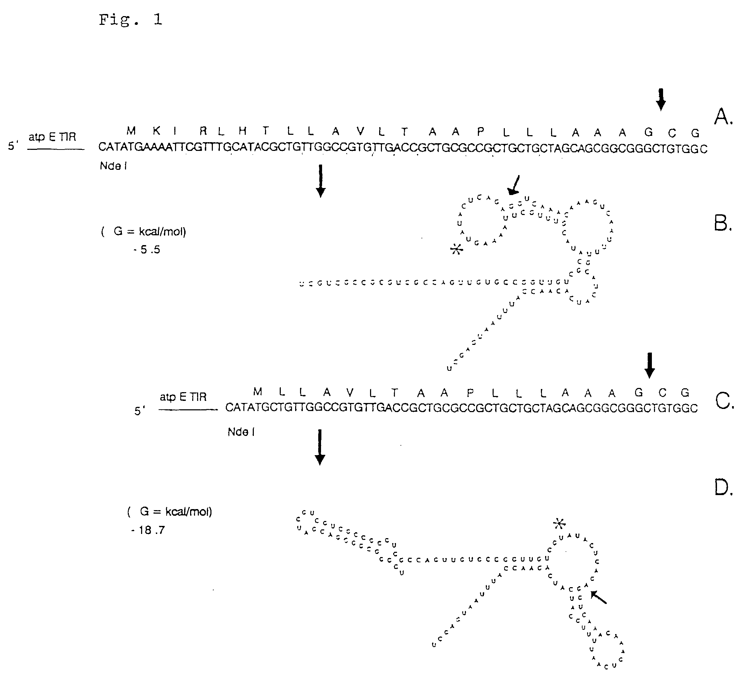

Gemäß einer Ausführungsform der Erfindung wird ein Hybrid-Plasmid zur Expression eines unfusionierten eine Prä-Sequenz aufweisenden etwa 38 kDa großen Antigens (Prä-Protein) von *M. tuberculosis* in E. *coli* vorgesehen, wobei das Plasmid
- eine DNA-Sequenz enthält, die 22 bis 17 Aminosäuren der folgenden Aminosäuresequenz kodiert: 
1         10         20
MKIRLHTLLAVLTAAPLLLAAAG
 und
- eine Restriktionsschnittstelle umfaßt, welche innerhalb ihrer Erkennungssequenz das Basentriplett ATG umfaßt, das das in Leserichtung erste Kodon (Methionin) der in Fig. 1A dargestellten Wildtyp-Präsequenz des etwa 38 kDa großen Antigens kodiert.

Dieses Hybrid-Plasmid kann dadurch gekennzeichnet sein, daß es sich bei dem etwa 38 kDa großen Antigen um ein Protein einer *M.-tuberculosis -Variante* handelt, die ebenfalls Tuberkulose hervorrufen kann.

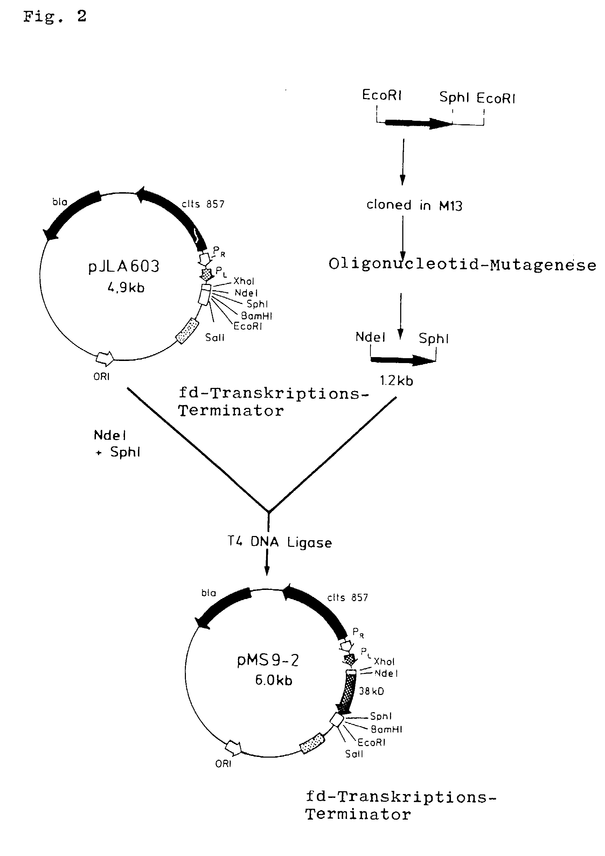

Ein erfindungsgemäßes Hybrid-Plasmid kann ferner dadurch gekennzeichnet sein, daß die das etwa 38 kDa große Antigen kodierende DNA-Sequenz mit ihrem 5'-Terminus in eine NcoI-, NdeI- oder SphI-Schnittstelle des Ausgangsvektors des Hybrid-Plasmids eingesetzt worden ist, beispielsweise pJLA603 (Abb.2) als Ausgangsvektor.

Ein erfindungsgemäßes Hybrid-Plasmid kann ferner durch eine die Präsequenz des etwa 38 kDa großen Antigens kodierende DNA-Sequenz gemäß Abb. 1 C gekennzeichnet sein.

Gemäß einer weiteren Ausführungsform betrifft die Erfindung *E. coli* mit einem erfindungsgemäßen Hybrid-Plasmid.

Gemäß einer weiteren Ausführungsform betrifft die Erfindung ein etwa 38 kDa großes Antigen von *M. tuberculosis,* herstellbar mit Hilfe von *E. coli* gemäß der Erfindung und folgendermaßen renaturierbar:
- Solubilisieren des als Einschlußkörper angefallenen Antigens und/oder Proteins in Guanidinium-HCl (gegebenenfalls in Gegenwart eines reduzierenden Agens), z. B. mit etwa 6 M Guanidinium-HCl und/oder in Gegenwart von Dithiothreit (DTT), und
- nachfolgendes Renaturieren mit Hilfe von Sephadex-Chromatographie, z. B. mit Hilfe von Sephadex G-25.

Gemäß einer weiteren Ausführungsform betrifft die Erfindung ein etwa 33 kDa großes M. tuberculosis-Protein, (i) herstellbar mit Hilfe von *E. coli* als Wirt von pJLA 603 als Expressions-Plasmid, wobei
- eine das etwa 38 kDa große Antigen von *M*. *tuberculosis* kodierende DNA-Sequenz mit ihrem 5'-Terminus in eine NcoI-Schnittstelle von pJLA 603 (Abb. 2) eingesetzt worden ist,
- diese eingesetzte DNA-Sequenz die Signalsequenz des Antigens umfaßt und
- die Erkennungssequenz der Schnittstelle das Basentriplett ATG umfaßt, das die in Leserichtung erste Aminosäure M (Methionin) kodiert, und
(ii) separierbar vom gleichfalls exprimierten etwa 38 kDa großen Antigen.

Erfindungsgemäß wurden rekombinante Plasmide konstruiert, welche in *Escherichia coli* in hohen Spiegeln das etwa 38 kDa große Antigen von *M. tuberculosis* produzieren. Mit den erfindungsgemäßen rekombinanten Konstrukten können große Mengen von unfusioniertem (einzigartigem) etwa 38 kDa großen Protein in *E. coli,* hauptsächlich in Form von Einschlußkörperchen hergestellt werden. Außerdem wurde im Rahmen der vorliegenden Erfindung ein Verfahren zur Isolierung und Reinigung des rekombinanten Antigens aus den Einschlußkörperchen entwickelt. Das auf diese Weise hergestellte, gereinigte etwa 38 kDa große Antigen ist immunologisch nicht unterscheidbar von dem nativen 38 kDa-Antigen von *M. tuberculosis.* Aufgrund seiner hohen Spezifität für *M. tuberculosis* kann dieses rekombinante Antigen weltweit nutzbringend zur serologischen Diagnostik der Tuberkulose eingesetzt werden. Das Antigen ist ebenso ein potentieller Kandidat für Impfstoffe gegen Tuberkulose, weil es immundominante T-Zell-Epitope umfaßt.

Das Gen, welches für das etwa 38 kDa große Antigen kodiert, wurde bereits kloniert und wird in der vorliegenden Erfindung als unfusioniertes Protein in *Escherichia coli* unter der Kontrolle starker transkriptionaler (Bakteriophage lambda P_{R}P_{L}) und translationaler *(atpE)* Signale exprimiert. Die Fermentation des *E. coli-Stammes* CAG629, dem eine lon-Protease und die Hitzeschockantwort fehlen, stellte das rekombinante Antigen in hohen Spiegeln zur Verfügung (etwa 10 % des gesamten Zellproteins). Das rekombinante Antigen, das als Einschlußkörperchen akkumulierte, wurde vollständig in 6 M Guanidiniumhydrochlorid solubilisiert, wieder gefaltet und erschien nach der Reinigung homogen. Das Produkt zeigte die erwartete Aminosäurenzusammensetzung und das erwartete Molekulargewicht, und ebenfalls gleichstarke Reaktivitäten mit drei verschiedenen monoklonalen Antikörpern wie das native Protein. Polyklonale Antikörper, die gegen das rekombinante Antigen entwickelt worden waren, reagierten in einem enzymgekoppelten Immunosorbent-Assay stark mit dem nativen Antigen. Diese Ergebnisse zeigen, daß das rekombinante etwa 38 kDa große Antigen, welches immunologisch nicht von dem nativen Protein aus *M. tuberculosis* unterschieden werden kann, in ausreichenden Mengen in *E*. *coli* produziert werden kann.

### Beschreibung der Abbildungen

- Abbildung 1:: Nukleotidsequenzen der 5'-Enden der konstruierten Gene, die die Prä-Form (A) des 38 kDA-Proteins und die verkürzte Form (C) des etwa 38 kDa großen Proteins kodieren. Die *Nde*I (CATATG)-schnittstelle wurde durch Oligonukleotid-Mutagenese unter Verwendung der Oligonukleotide 5'-AGCACAGAAAGGTATCATATGAAAATTCGTTTGCATA-3' (für A) und 5'-ATTCGTTTGCATATGCTGTTGGCCGTGT-3' (für C) geschaffen. Die abgeleitete Aminosäuresequenz ist in dem Ein-Buchstaben-Code dargestellt und der Pfeil oberhalb der Sequenz zeigt die vermutete Prozessierungsstelle. Die RNA-Stabilität in der Region des ATG-Startcodons der Prä-Form (B) und der verkürzten Form (D) wurden nach der Methode von Zucker und Stiegler berechnet (28). Die Ribosombindungsstelle auf dem *atp*E-Gen und das Initiationscodon des Antigengens von 38 kDa (Abb. 1B) bzw. etwa 38 kDa (Abb. 1D) sind mit einem Stern beziehungsweise mit einem Pfeil markiert.
- Abbildung 2:: Konstruktion eines nicht-erfindungsgemäßen Expressionsplasmids. Ein 2.0 kb *Eco*RI-Fragment, welches das Gen für das 38 kDa-Protein umfaßt, wurde aus Lambda AA59 (3) in M13mp19 kloniert und mutagenisiert, um eine *Nde*I (CATATG)Schnittstelle auf eine solche Weise zu schaffen, daß diese das Initiationscodon des Gens beinhaltet. Ein 1.2 kb *Nde*I-*Sph*I-Fragment wurde dann in die *Nde*I*-Sph*I-Schnittstelle des Expressionsvektors, pJLA603, subkloniert, der die Promotoren P_{R} und P_{L} des Bakteriophagen Lambda in einer Tandemanordnung, die *atp*E-Translationsinitiationsregion (weißer Kasten), den transkriptionellen Terminator fd und translationale Stopcodons in allen drei Leserastern enthält (20). Die Plasmide sind nicht maßstabsgetreu abgebildet.

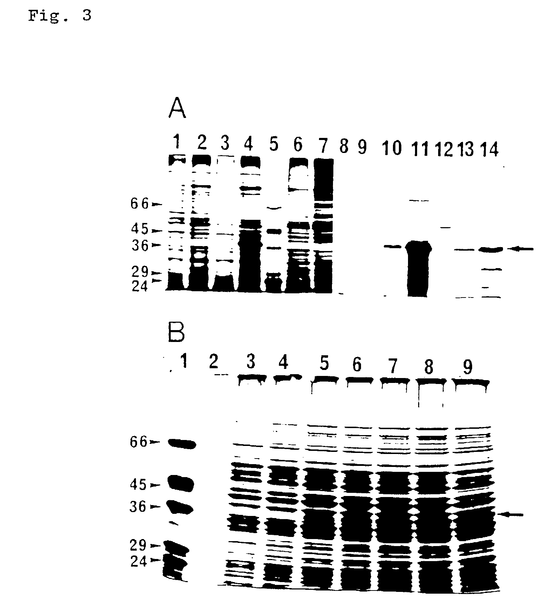

- Abbildung 3:: Expression von rekombinanten Proteinen von 38 kDa und etwa 38 kDa in *E. coli* CAG629 in Batchkulturen (A) und in einem Bioreaktor (B). A: Silbergefärbte Gele (Spuren 1-7) und die korrespondierenden Immunoblots mit MAb HBT12 (Spuren 8-14), welche die Proteine zeigen, die von nicht-erfindungsgemäßen CAG629 (pMS9-2) nach Wachsen bei 30°C (Spuren 1/8 und 2/9) und bei 42°C (Spuren 3/10 und 4/11) produziert wurden. Die Proteinmuster von erfindungsgemäßen CAG629 (pMS10-4) nach Induktion bei 42°C sind in Spur 6/13 und 7/14 gezeigt. Spuren 1, 3 und 6 repräsentieren die löslichen Fraktionen und Spuren 2, 4 und 7 die Einschlußkörperchen. Spur 5: Molekulargewichtsmarker (die Werte sind links in Kilodaltons angegeben), 12: vorher gefärbter Standard.
B: Akkumulation des rekombinanten 38 kDa-Proteins von nicht-erfindungsgemäßen CAG629 (pMS9-2) in dem Bioreaktor bei 30°C (Spur 3) und nach Induktion bei 42°C (Spuren 4-9). Proben wurden alle 30 Minuten nach Induktion gezogen. Spur 1 zeigt den Molekulargewichtsmarker und Spur 2 kleine Mengen des 38 kDa-Proteins, das aus Einschlußkörperchen aus einer Batchkultur stammt. Der Pfeil zeigt auf die Doppelbande, die mit dem rekombinanten Produkt korrespondiert.
- Abbildung 4:: Elektronenmikroskopische Abbildung von Schnitten von CAG629 (pMS9-2), die Einschlußkörperchen zeigt (Pfeile).
- Abbildung 5:: SDS-12.5 %-PAGE-(Silberfärbung)-Analyse verschiedener Reinigungsschritte. Spuren 1 und 4 zeigen die Molekulargewichtsstandards. Spur 2: solubilisierte Einschlußkörperchen; Spur 3: Proteine, die während des Waschens der Einschlußkörperchen vor der Solubilisierung freigesetzt wurden; Spur 5: Proteine, renaturiert auf Sephadex G25; Spuren 6-7: QAE-Sepharose-Eluate. Der Pfeil zeigt das rekombinante 38 kDa-Protein von nicht-erfindungsgemäßen CAG629(pMS9-2).

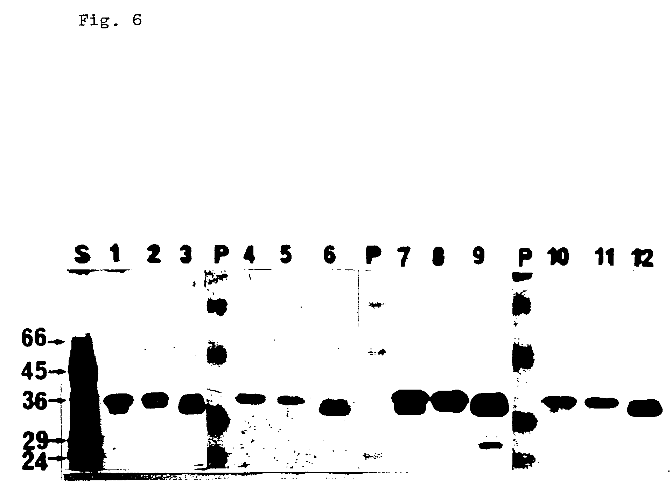

- Abbildung 6:: SDS-PAGE- und Immunoblotanalysen der FPLC-gereinigten 38 kDa-Proteinpräparationen von nicht-erfindungsgemäßen CAG629 (pMS9-2). Proben, die etwa 1 µg Protein enthielten, wurden mittels SDS-12.5 %-PAGE aufgetrennt und entweder silbergefärbt (Spuren 1-3) oder unter Verwendung von anti-38 kDa-MAbs immunogeblottet: HAT2 (Spuren 4-6), HBT12 (Spuren 7-9) und HYT28 (Spuren 10-12). Das rekombinante Protein, das mit den drei MAbs reagierte, wurde in Hauptpeaks gefunden: einem bei 100 mM NaCl (Spuren 1, 4, 7 und 10), und dem anderen bei 130-200 mM NaCl (Spuren 3, 6, 9 und 12). Spuren 2, 5, 8 und 11 zeigen das Protein, das in Gegenwart von Triton X-100 gereinigt wurde (eluiert bei 170 mM NaCl). Der Molekulargewichtsstandard wurde in Spur S aufgetragen (die Größen sind in Kilodaltons an der linken Seite angegeben), und die Spur P repräsentiert den vorher gefärbten, während des Immunoblots verwendeten Marker.
- Abbildung 7:: Vergleich des nativen und des rekombinanten 38 kDa-Antigens von nicht-erfindungsgemäßen CAG629 (pMS9-2).
A: Etwa 1 µg des mittels Affinitätschromatographie gereinigten nativen Proteins (Spuren 1 und 3) und des rekombinanten Proteins, Präparation-I (Spuren 2 und 4) wurden durch SDS-12.5 % PAGE aufgetrennt und entweder silbergefärbt (Spuren 1 und 2) oder unter Verwendung des MAb's HBT12 immunogeblottet (Spuren 3 und 4). Spur P zeigt den vorher gefärbten Molekulargewichtsstandard,
B: Laserdensitometrischer Vergleich der Immunreaktivität des nativen und des rekombinanten 38 kDa-Proteins von nicht-erfindungsgemäßen CAG629 (pMS9-2). Das silbergefärbte Gel und der Immunoblot aus Teil (A) wurden mit einem Laserdensitometer abgetastet. Die Peakflächen des silbergefärbten nativen (Nat. Silver) und des rekombinanten Proteins (Rec. Silver) ebenso wie jene des immunogeblotteten nativen (Nat. Immuno) und des rekombinanten Proteins (Rec. Immuno) sind dargestellt.

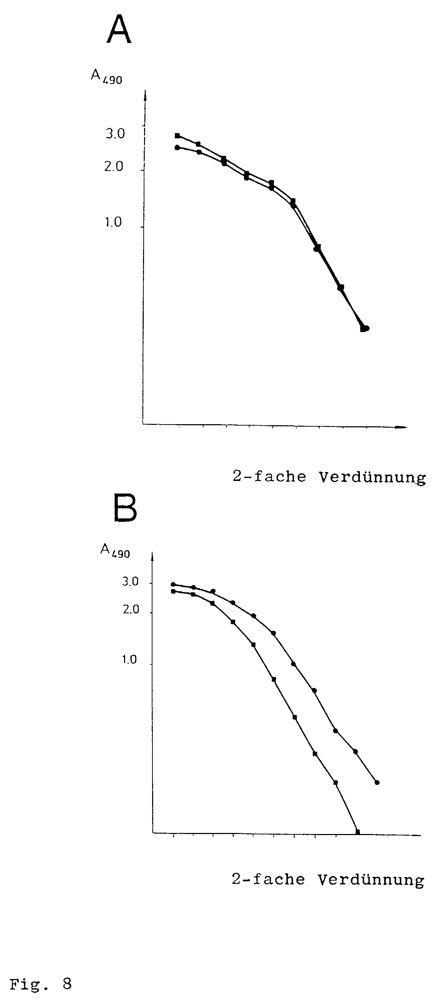

- Abbildung 8:: A: Titrationskurven von polyklonalen Sera mit dem 38 kDa-Antigen von nicht-erfindungsgemäßen CAG629 (pMS9-2). Sera von Kaninchen, die mit dem nativen (Punkt) oder dem rekombinanten 38 kDa-Protein (ausgefülltes Kästchen) immunisiert wurden. Serielle zweifache Verdünnungen, beginnend mit einer 1:100-Verdünnung, wurden in Mikrotiterplatten titriert, die mit der Präparation I des rekombinanten 38 kDa-Proteins beschichtet worden waren (0.1 µg/Vertiefung). Die gebundenen Immunglobuline wurden mittels an anti-Kaninchen-Immunglobuline vom Schwein gekoppelte Meerrettichperoxidase in 1:1000-facher Verdünnung (P 217, Dakopatts, Glostrup, DK) nachgewiesen.
B: Wie (A) mit der Ausnahme, daß die Mikrotiterplatten mit dem nativen 38 kDa-Antigen beschichtet worden waren (0,1 µg/Vertiefung).

Im folgenden wird die Erfindung im Detail erläutert.

### Material und Methoden

### Bakterienstämme, Phagen, Plasmide und Wachstumsbedingungen.

Die in dieser Erfindung verwendeten E. coli-Stämme waren TG-1 (Δlac-pro, *sup*E*, thi, hsd*D5/F'*tra*D36, proA⁺ B⁺, *lac*I^{q}, *lac*Z ΔM15), DH5alpha (*end*A1, *rec*A1, *hsd*R17, *sup*E44*, thi*-1, *gyr*A96, *rel*A1, (*lac*ZYA-*arg*F), φ 80d/*lac*Z ΔM15), EC 538 und CAG629 (*lon, htp*R165-Tn*10*; C. Gross). Der rekombinante Lambda gt11-Bakteriophage, Klon AA59, wurde in einer früheren Untersuchung (3) aus einer genomischen DNA-Bibliothek von M. *tuberculosis* (konstruiert von R. A. Young (27)) isoliert.

Wenn nicht anderweitig angegeben, wurden die Stämme in Luria-Bertani-Medium (15) bei 37°C kultiviert. Die flüssigen Kulturen wurden mit Sauerstoff versorgt, indem sie mit 160 Upm in einem Pilot-Shake-Schüttler (Kühne, Schweiz) umgewälzt wurden.

### DNA-Manipulationen.

Präparation und Behandlung der DNA wurden gemäß standardisierten Protokollen ausgeführt (15). Die Transformation wurde wie bei Hanahan beschrieben bewerkstelligt (8). Die DNA-Sequenzierung wurde mittels Dideoxynukleotid-Kettenabbruchmethode durchgeführt (18). Oligonukleotide wurden auf einem DNA-Synthesizer von Applied Biosystems, Modell 380B, hergestellt und über OPC-Säulen (Applied Biosystems Inc.) gereinigt.

### Oligonukleotid-Mutagenese.

Das 2,0 kb EcoRI-Fragment aus dem genomischen Klon Lambda-AA59 (3) wurde in M13mp19 transferiert. Die Präparation von einzelsträngiger DNA und die Oligonukleotid-Mutagenese wurden unter Verwendung des Amersham-Kits (RPN 1523) durchgeführt. Die DNA-Sequenz des Oligonukleotids vor und nach Mutagenisierung wurden durch DNA-Sequenzierung (18) bestätigt.

### Präparation eines groben Proteinextrakts in kleinem Maßstab.

Die Stämme wurden in LB-Medium, supplementiert mit Ampicillin (100 µg/ml), bei 30°C bis zu einer Absorption von 0.6 bei 580 nm kultiviert. Die Kulturen wurden durch dreistündige Temperaturerhöhung auf 42°C in einem Schüttelwasserbad induziert. Bakterien aus 1 ml Kulturmedium wurden geerntet, in 100 µl Probenpuffer (62 mM Tris-HCl, pH 6.8; 2 % Natriumlaurylsulfat; 0.7 M 2-Mercaptoethanol; 10 % Glycerol; 0.002 % Bromphenolblau) suspendiert und durch Behandlung mit Ultraschall auf Eis (3 x 30 Sekunden; 50 W) unter Verwendung eines Braun Labsonic 2000 aufgebrochen. Die Proben wurden 10 Minuten lang auf 95°C erhitzt und 10 µl wurden mittels Polyacrylamidgelelektrophorese analysiert.

### Kultivierung im Bioreaktor.

30 l eines modifizierten konzentrierten LB-Mediums (Trypton 40 g/l; Hefeextrakt 20 g/l; NaCl 5 g/l) wurden in Gegenwart von 3.5 ml Ucolub N38 Antifoam (Brenntag, Mülheim, FRG) in einem 50 l Bioreaktor (Biostat U30D, Braun Melsungen, FRG) sterilisiert. Die Kultivierung wurde durch Inokulation mit 0.5 l einer Kultur der Organismen, die über Nacht in dem selben Medium gewachsen waren, gestartet, wobei die initiale Absorption A₅₄₆ = 0.04 betrug. Die Rührgeschwindigkeit wurde konstant bei 300 Upm belassen, was die Konzentration an gelöstem Sauerstoff nahe der Sättigung hielt, und der pH wurde bei 6.9 eingestellt. Nach 4 Stunden Fermentationszeit (A₅₄₆ = 0.4) wurde die Temperatur von 30°C auf 42°C erhöht und bei dieser Temperatur weitere 4 Stunden lang belassen. Am Ende der Induktionsperiode wurde die Suspension in einem geschlossenen System mittels Scherstrom-Mikrofiltration (Enka-Modul Typ A7 ABA 3A) mit einer 0.23 m² Accural-Membran (0.2 µm Porendurchmesser; Enka, Wuppertal, FRG) konzentriert, bis ein Endvolumen von 10 1 erhalten wurde.

### Proteinreinigung.

Die aus dem Bioreaktor gewonnenen Zellen wurden aufgebrochen, indem man sie einmal bei 500 bar mit einer Flußrate von 60 l/h durch einen Hochdruck-Homogenisierer LAB 60/500/2 (A.P.V.-Schröder, Lübeck, FRG) passieren ließ. Die Einschlußkörperchen wurden grob unter Verwendung eines Zentrifugalseparators SA 1-01-175 (Westfalia, Oelde, FRG) abgetrennt. Diese Vorrichtung erlaubt die Isolierung von
Einschlußkörperchen aus dem Medium mit einer Flußrate von 15-20 l/h. Die Einschlußkörperchen wurden zweimal mit 200 ml Puffer L (50 mM Tris-HCl, 10 mM EDTA, pH 8.0), der 2 % Triton X-100 enthielt, gewaschen. Die gewaschenen Niederschläge wurden unter langsamem Rühren in 3 1 Puffer L, der 6 M Guanidiniumhydrochlorid und 20 mM DTT enthielt, über 16 Stunden bei 4°C resuspendiert. Nach Zentrifugation bei 7000 g wurde der flüssige Überstand auf eine Sephadex G-25-Gelfiltrationssäule (10 x 90 cm) aufgetragen, die mit 10 mM Tris-HCl-Puffer, pH 7.0, der 100 mM NaCl enthielt,
äquilibriert worden war, um das Guanidinium-HCl zu entfernen und um die Renaturierung des rekombinanten Antigens zu bewerkstelligen. Die Lösung wurde in 2 l-Aliquots mit einer Flußrate von 7 l/h aufgetragen und in dem Eluat wurden O.D.₂₆₀ und Leitfähigkeit gemessen. Die entsalzte und renaturierte Antigenlösung wurde in dem Startpuffer der Leitfähigkeit von 8 mS/cm gefunden, gut getrennt von dem Salzpeak, der hauptsächlich Guanidinium-HCl enthielt. Die Antigenpeaks wurden vereinigt und mit destilliertem Wasser verdünnt, um eine Leitfähigkeit von 5 mS/cm zu erreichen, und der pH wurde mit 1 M Tris auf 8.5 eingestellt. Diese Lösung wurde für die folgenden Reinigungsschritte in zwei Teile geteilt.
Ein Teil der Lösung wurde auf eine FPLC-Säule (QAE-Sepharose FF; 5 x 18 cm) gegeben, die mit einem 20 mM Tris-HCl-Puffer, pH 8.0 äquilibriert worden war. Die Flußrate betrug 1.72 l/h, korrespondierend zu einer linearen Flußrate von 86.6 cm/h. Nach extensivem Waschen mit Startpuffer wurde das Antigen durch Auftragen eines gestuften Gradienten, der aus 50 mM, 100 mM, 250 mM, 500 mM und 1 M Natriumchlorid in Startpuffer bestand, eluiert. Anschließend wurde die Säule mit Startpuffer re-äquilibriert, der zweite Teil aus der Gelfiltrationssäule aufgetragen und wie oben beschrieben eluiert. Die antigenenthaltenden Fraktionen der zwei QAE-Sepharose-Läufe wurden vereinigt und mittels Ultrafiltration unter Verwendung einer Amicon Hohlfaser-Vorrichtung (Typ H1P10; Ausschlußgrenze 10.000) konzentriert und diafiltriert (2 mS/cm). Etwa 20 mg des so erhaltenen Proteins wurden auf eine Mono Q HR (5/5)-FPLC-Säule, die mit 20 mM Tris-HCl, pH 8.0 äquilibriert worden war, gegeben. Die Säule wurde mit Startpuffer bei einer Flußrate von 2 ml/min und einem Druck von 25 bar gewaschen. Danach wurde das Antigen durch schrittweise Erhöhung der NaCl-Konzentration bis auf 0.5 M in Startpuffer eluiert. Insgesamt wurden drei Mono Q HR (5/5)-Läufe unter den oben beschriebenen Bedingungen durchgeführt.

### Polyacrylamidgelelektrophorese und Immunoblotting.

Die grob gereinigten und hochgereinigten Proteine wurden mittels Natriumlaurylsulfat/Polyacrylamid (12 %)-Gelelektrophorese (11) analysiert. Die Proteinproben wurden mit einem 2 x-Probenpuffer im Verhältnis 1:1 gemischt und 10 Minuten lang auf 95°C erhitzt, bevor man sie auf das Gel auftrug. Im Anschluß an die Elektrophorese wurden die Polypeptide durch Silberfärbung sichtbar gemacht (5). Die Proteinkonzentrationen wurden nach der Methode von Lowry et al. gemessen (13). Die Proteine wurden mittels einer selbst gefertigten halbtrockenen Blottingapparatur unter Verwendung von 25 mM Tris, 192 mM Glycin, 20 % Methanol, pH 7.4, auf eine Nitrocellulosemembran (BioRad) transferiert. Eine unspezifische Bindung wurde durch Inkubation des Filters mit TBS (50 mM Tris-HCl; 200 mM NaCl, pH 7.5), das eine 10 %ige Milchlösung (0.3 % Fett) enthielt, blockiert. Die primären Antikörper (monoklonale Antikörper aus Mäusen) wurden 1000-fach in TBS verdünnt und die Filter damit über Nacht bei 4°C inkubiert. Die Filter wurden 3 Mal mit TBS gewaschen und die Immundetektion wurde mit einem biotinyliertem anti-Maus-IgG und einem Konjugat aus Streptavidin und alkalischer Phosphatase (BRL, Gaithersburg, MD, USA) ausgeführt. Für einen Immuno-Dot-Blot-Assay wurden die Proteinproben durch eine Nitrocellulosemembran unter Verwendung eines BioRad Bio-Dot-Apparates gefiltert und dann wie oben beschrieben behandelt. Die monoklonalen Antikörper HAT2, HBT12 und HYT28 wurden früher beschrieben (2, 12, 21). Densitometrische Messungen der silbergefärbten Gele und der Western Blots wurden mit einem Laserdensitometer (LKB) vorgenommen.

### Aminosäureanalyse.

Die Aminosäureanalysen wurden mit einem Biotronik LC-5001 Aminosäureanalyser (Maintal, FRG) durchgeführt, nachdem die Proteinprobe in 6 N HCl, die 0.1 % Phenol enthielt, über 24 Stunden bei 105°C hydrolysiert worden war.

### Herstellung der polyklonalen anti-38 kDa-Protein-Sera.

Kaninchen wurden subkutan entweder mit affinitätschromatographisch gereinigtem nativen 38 kDa-Protein (25) oder mit rekombinantem 38 kDa-Protéin (Präparation I) von nicht-erfindungsgemäßen CAG629 (pMS9-2) immunistiert. Das Antigen (10 µg/Dosis) wurde an Aluminiumhydroxid (2.4 mg) adsorbiert und nachfolgend mit 1 ml Freund's inkomplettem Adjuvans gemischt. Die Kaninchen wurden drei Mal mit Intervallen von zwei Wochen immunisiert. Blut wurde 10 Tage nach der letzten Immunisierung gewonnen und die IgG-Fraktion nach der Methode von Harboe und Inglid (9) gereinigt.

### Elektronenmikroskopie.

Die Zellen wurden mit 1 % Formaldehyd und 0.2 % Glutaraldehyd in PBS (50 mM K-Phosphat, 0.9 % NaCl, pH 6.9) eine Stunde lang auf Eis fixiert. Nach mehrmaligem Waschen mit PBS wurden die Zellen gemäß der progressiven Temperaturerniedrigungsmethode (PLT), (17), eingebettet. Die Zellen wurden zuerst mit 10 %, dann mit 30 % Ethanol über 30 Minuten auf Eis, anschließend mit 50 % Ethanol über 30 Minuten bei -20°C, und schließlich mit 70 %, 90 % und 100 % Ethanol bei -35°C jeweils 30 Minuten lang und mit 100 % Ethanol bei -35°C 1 Stunde lang dehydratisiert. Die Infitration mit dem Lowicrylharz K4M wurde wie folgt ausgeführt: 1 Teil Ethanol/1 Teil K4M-Harz über Nacht bei -35°C, 1 Teil Ethanol/2 Teilen K4M-Harz 12 Stunden lang und reines K4M-Harz 2 Tage lang bei -35°C mit mehrmaligem Wechseln der Harzmischung. Die Polymerisierung des Harzes wurde durch UV-Licht (366 nnm) über 1 Tag bei -35°C und bei Raumtemperatur über weitere 2 Tage erreicht. Ultradünnschnitte wurden mit Uranylacetat und Bleicitrat nachgefärbt, bevor man sie mit einem Zeiss EM 10B Transmissionselektronenmikroskop bei einer Beschleunigungsspannung von 80 kV untersuchte.

### Ergebnisse

### Konstruktion der Expressionsplasmide.

Die DNA-Sequenzierung des Gens des nativen 38 kDa-Proteins ergab ein offenes Leseraster, das ein Polypeptid mit 374 Aminosäuren kodierte und GTG als das Startcodon enthielt (1). Ebenso fand sich eine 24 Aminosäuren lange Signalsequenz (Abbildung 1A), die Ähnlichkeiten mit jenen von bakteriellen Lipoproteinen aufwies. In der vorliegenden Erfindung wurde das Gen manipuliert, so daß in dem Expressionsvektor pJLA603 (Abbildung 2) in hohem Ausmaß ein unfusioniertes 38 kDa-Antigen exprimiert werden konnte. Zur Klonierung und Expression in derartigen Vektoren ist es erforderlich, daß das fremde Gen eine Restriktionsschnittstelle aufweist, zum Beispiel *Nco*I, *Nde*I, *Sph*I, welche innerhalb ihrer Erkennungssequenz ATG im Leseraster umfaßt. Weil sich keine derartige Schnittstelle in der Region um das Initiationscodon des Gens des nativen 38 kDa Proteins befindet, wurde eine Oligonukleotid-Mutagenese in M13mp19 durchgeführt, um einen Austausch des Startcodons von GTG zu ATG und eine *Nde*I-Schnittstelle am N-Terminus vorzusehen (Abbildungen 1 und 2). Das 1.2 kb *Nde*I-*Sph*I-Fragment, das aus dem M13-Derivat nach Mutagenisierung herausgeschnitten werden konnte, wurde dann zwischen die *Nde*I-*Sph*I-Schnitt- stellen von pJLA603 kloniert. Das nicht erfindungsgemäße rekombinante Plasmid, welches so konstruiert war, daß ein rekombinantes 38 kDa-Protein mit seinem ursprünglichen, intakten Signalpeptid exprimiert werden konnte, wurde pMS9-2 genannt. In gleicher Weise wurde ein anderes erfindungsgemäßes rekombinantes Plasmid, pMS10-4, welches eine Deletion der ersten 6 Aminosäuren in der Signalsequenz aufwies, hergestellt (Abbildung 1C). Eine Computeranalyse der Translationsinitiationsregion der von nicht-erfindungsgemäßern pMS9-2 spezifizierten m-RNA sagte eine lockere Sekundärstruktur voraus (Abbildung 1B), welche für eine hochgradige Expression in *E*. *coli* sehr vorteilhaft sein sollte (14). Auf der anderen Seite zeigte erfindungsgemäßes pMS10-4 eine stabilere Sekundärstruktur (Abbildung lD), was darauf hindeutet, daß die Expression dieses Plasmids nicht so gut sein würde wie die von nicht-erfindungsgemäßern pMS9-2. Beide rekombinanten Plasmide wurden für die Expressionsuntersuchungen herangezogen.

### Expression des rekombinanten Antigens in einer Kultur im kleinen Maßstab.

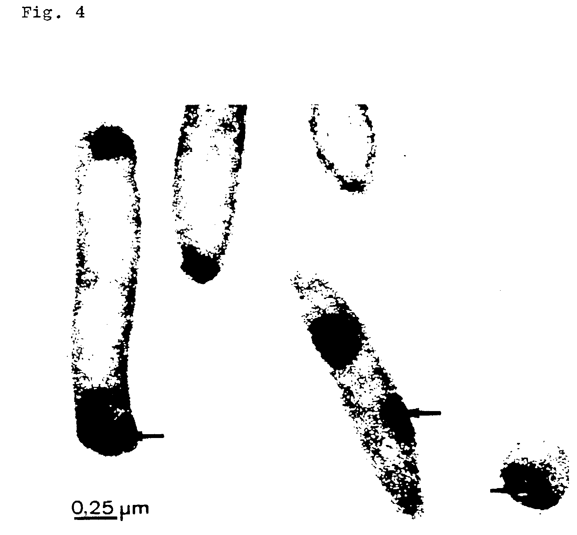

Mehrere *E. coli-Stämme* (DH5α; EC538 und CAG629) wurden im Hinblick auf die Expression von rekombinanten Antigenen von 38 bzw. etwa 38 kDa, kodiert durch pMS9-2 und pMS10-4, untersucht. Der *lon, htp*R-Stamm CAG629 zeigte die stärkste Expression. Extrakte aus nicht-erfindungsgemäßen CAG629 (pMS9-2)-Zellen, die bei 42°C induziert worden waren, enthielten ausreichende Mengen des rekombinanten Proteins (Abbildung 3, Spur 3 und 4), wogegen es in Extrakten aus uninduzierten Zellen nicht auffindbar war (Abbildung 3, Spur 1). Der nicht-erfindungsgemäße rekombinante Stamm zeigte keine offensichtlichen Störungen nach Induktion und wuchs fortgesetzt und exponentiell (Daten nicht gezeigt). Immunoblotting mit MAb's HBT12 (Abbildung 3A, Spuren 10 und 11), HAT2 und HYT28 (Daten nicht gezeigt) ergaben positive Reaktionen mit dem rekombinanten 38 kDa-Protein. Der größte Anteil des rekombinanten Proteins fand sich in der Zellpellet-Fraktion der aufgebrochenen Zellen, und nur ein kleiner Anteil war in dem flüssigen Überstand nachweisbar (Abbildung 3A, Spuren 10 und 11). Der erfindungsgemäße rekombinante Klon CAG629 (pMS10-4) produzierte, wie nach der Vorhersage der Sekundärstruktur zu erwarten war, beträchtlich weniger Protein als nicht-erfindungsgemäßes pMS9-2 (Abbildung 3A, Spuren 13 und 14). Die auf dem Immunoblot erkennbaren schwachen Banden, die langsamer wanderten als das 38 kDa-Protein, korrespondieren zu SDS-unlöslichen, aggregierten Formen des rekombinanten Proteins.
Das rekombinante 38 kDa-Protein wurde in hohem Ausmaß (etwa 10 % des gesamtem Zellproteins) produziert, wie mittels Messungen mit dem Laserdensitometer an silbergefärbten SDS-PAGE-Gelen gemessen werden konnte. Wie es oft bei rekombinanten Proteinen der Fall ist, die in hohem Ausmaß in *E. coli* produziert werden (7, 19), ist das 38 kDa-Protein hauptsächlich in cytoplasmatischen Aggregaten oder Einschlußkörperchen vorhanden (Abbildung 4).

### Fermentation des rekombinanten E. coli und Reinigung des rekombinanten Antigens.

Der nicht-erfindungsgemäße rekombinante Klon CAG629 (pMS9-2) wurde für eine 30 l-Fermentation ausgewählt, weil er in Batchkulturen die höchsten Antigenspiegel produzierte und tolerierte und weil er ein 38 kDa-Protein mit intakter Signalsequenz kodierte. Der zeitliche Verlauf der Produktion von rekombinantem Antigen in dem Bioreaktor wurde beobachtet (Abbildung 3B). Eine SDS-PAGE des gesamten Zellextrakts zeigte, daß binnen 30 Minuten nach Erhöhung der Temperatur von 30°C auf 42°C eine Doppelbande von 38 kDa Größe auf silbergefärbten Gelen klar erkennbar war.

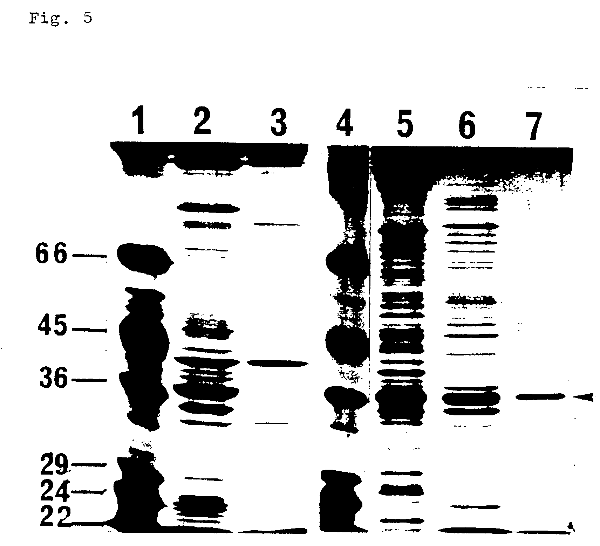

Das Waschen der Einschlußkörperchen, die aus der Fermenterkultur erhalten wurden, mit Puffer, der Triton X-100 enthielt, bewirkte eine Entfernung einiger verunreinigender Proteine (Abbildung 5, Spur 3) ohne einen augenscheinlichen Verlust an rekombinantem Antigen (Abbildung 5; Spur 2). Unter Verwendung einer Sephadex G-25-Säule wurde das Antigen entsalzt und renaturiert (Abbildung 5, Spur 6); wir beobachteten auf dieser Stufe keine Reaggregation des Antigens. Weitere Reinigung des Antigen wurde durch mehrere Zyklen einer FPLC-Anionenaustauschchromatographie erreicht (Abbildung 6), bei der gefunden wurde, daß das Antigen bei 100 mM NaCl (Präparation I) und zwischen 130-200 mM NaCl (Präparation III) eluierte. Die in Abbildung 6 gezeigte Präparation II repräsentiert das Antigen, das über eine FPLC-Anionenaustauschersäule in Gegenwart von Triton X-100 aus den aggregierten und verunreinigten Fraktionen vorhergehender Anionenaustauschchromatographieschritte gereinigt wurde.

### Struktur, immunologische Reaktion und Immunogenität des gereinigten, rekombinanten Proteins.

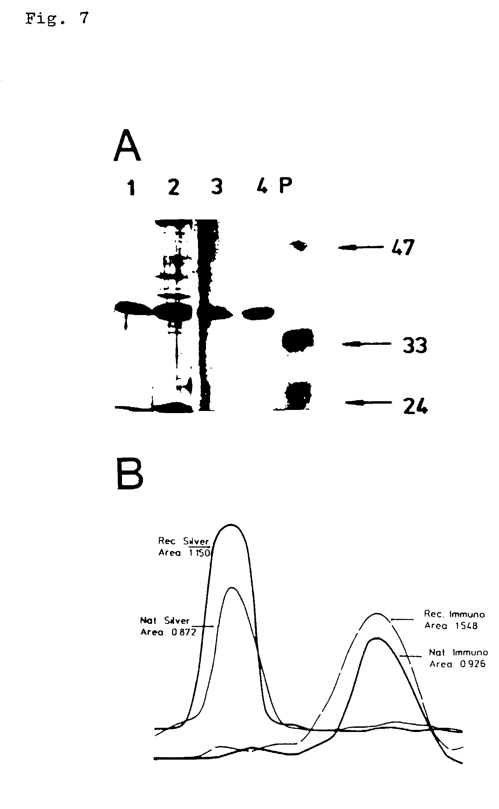

Die immunologische Reaktion der gereinigten Antigenpräparationen wurde mit den monoklonalen Antikörpern HAT2, HBT12 und HYT28 getestet. Alle drei Antikörper reagierten mit den rekombinanten Antigenpräparationen (Abbildung 6). Wenn das affinitätschromatographisch gereinigte native Antigen mit dem rekombinanten Antigen (Präparation I) mittels SDS-PAGE und Immunoblot verglichen wurde, ergaben sich keinerlei Unterschiede (Abbildung 7A). Das silbergefärbte Gel und der Immunoblot aus Abbildung 7A wurden auch mit einem Laserdensitometer analysiert, und wir beobachteten, nach Normalisierung im Hinblick auf Unterschiede in den aufgetragenen Proteinmengen, daß das native und das rekombinante Antigen identische Reaktionen mit den monoklonalen Antikörpern HBT12 (Abbildung 7B), HAT2 und HYT28 (Daten nicht gezeigt) ergaben.

Weiter wurde die Immunogenität des rekombinanten Antigens getestet, indem man aus Kaninchen polyklonale Antisera gegen das native und das rekombinante Antigen unter ähnlichen Bedingungen gewann. Die beiden Seren wurden nachfolgend im enzymgekoppelten Immunosorbent Assay (ELISA) sowohl gegen das rekombinante (Abbildung 8A) als auch das native Antigen (Abbildung 8B) getestet. Die Steilheit der Kurven ist identisch, was zeigt, daß die beiden Seren das native und das rekombinante 38 kDa-Protein gleich gut binden.

Die Aminosäurenzusammensetzung des rekombinanten 38 kDa-Proteins gibt ziemlich gut die Aminosäurenkomposition wieder, die sich aus der Nukleotidsequenz ableiten läßt (Tabelle 1).

**Etwa 33 kDa großes Protein**. Die Kürzung im Vergleich zum etwa 38 kDa-großen Antigen hat die Signalsequenz völlig entfernt. Jedoch reagiert das gekürzte Protein stark mit den monoklonalen Antikörpern HAT2, HBT12 und HYT28, was zeigt, daß die drei Epitope intakt sind. Das gekürzte Protein zeigt auch eine starke Reaktion im ELISA- und im Western-Blotting-Test gegenüber Seren von Mäusen, die mit *Mycobacterium tuberculosis* infiziert worden waren. Im Rahmen der vorliegenden Erfindung wurde festgestellt, daß etwa 85 % der Seren von Tuberkulose-Patienten positiv mit dem gekürzten Protein bzw. dem gekürzten Antigen reagierten, was zeigt, daß das gekürzte Antigen wirksam zur Diagnose eingesetzt werden kann.

### Diskussion

Es wurde eine Strategie entwickelt und ausgeführt, ein DNA-Fragment von *Mycobacterium tuberculosis* in einen Expressionsvektor zu klonieren, so daß unfusioniertes etwa 38 kDa großes Protein in hohem Ausmaß produziert werden würde. Der Vektor umfaßte den Lambda-P_{R}P_{L}-Promotor und die effiziente Translationsinitiationsregion des *atp*E-Gens, was eine Expression des heterologen etwa 38 kDa großen Antigens in *E*. *coli* in einem Ausmaß bewirkte, das 10 % des gesamten Zellproteins entsprach. Etwa 15 mg rekombinantes Protein/Liter wurde unter diesen gegebenen Bedingungen hergestellt. Es sollte allerdings betont werden, daß das Ziel der vorliegenden Erfindung war, herauszufinden, ob das überexprimierte rekombinante Antigen in einer antigenen Form gefunden werden konnte, die immunologisch nicht unterscheidbar von dem nativen Antigen ist, das man aus *Mycobacterium tuberculosis* gewinnt, und nicht, die Fermentationsausbeuten zu optimieren.

Der Hauptanteil des rekombinanten etwa 38 kDa großen Proteins akkumulierte in Form von Einschlußkörperchen. 6 M Guanidinium-HCl in Gegenwart eines reduzierenden Agens (DTT) in hoher Konzentration stellte sich als am besten geeignet für die Solubilisierung der Einschlußkörperchen heraus. Die Renaturierung der Proteine der Einschlußkörperchen, insbesondere der hydrophoben Membranproteine, ist oft schwierig und es bedarf einer sorgfältigen Optimierung der experimentellen Bedingungen. In unserem Fall bewirkte das übliche Verfahren der Dialyse oder schrittweisen Dialyse eine Präzipitation des rekombinanten etwa 38 kDa großen Proteins, selbst bei sehr niedrigen Proteinkonzentrationen (0.05 mg/ml). Die Renaturierung des rekombinanten Proteins auf Sephadex G-25 stellte sich auf anderen Seite als effektiv heraus, und es wurde keine signifikante Reaggregation des Proteins beobachtet. Beginnend mit den Einschlußkörperchen, die etwa 200 mg Gesamtprotein enthalten, konnten wir 19 mg des etwa 38 kDa großen Proteins mit einer Reinheit von größer 95 % darstellen, wie an silbergefärbten SDS-PAGE-Gelen gezeigt wurde.

Das native 38 kDa-Antigen aus *M*. *tuberculosis* ist höchstwahrscheinlich ein Lipoprotein (D.B. Young und T.R. Garbe, Res. Microbiol. 142:- (1991), im Druck). Lipoproteine zeigen aberrante, diffuse Banden auf SDS-PAGE-Gelen (16, 22). Wie es auch für andere Lipoproteine gilt, beobachteten wir eine deutliche Tendenz des rekombinanten etwa 38 kDa großen Antigens zur Aggregation während der Anionenaustauschchromatographie und während der Konzentrierung durch Ultrafiltration. Ein Teil des Antigens, welcher während der Ultrafiltration aggregierte, konnte durch Solubilisierung in 2 % Triton X-100, gefolgt von FPLC auf Mono Q, wiedergefunden werden.

Das native 38 kDa-Protein erscheint auf SDS-Polyacrylamidgelen als Doppelbande. Der Grund hierfür ist nicht bekannt, er könnte jedoch von einer Acylierung und Prozessierung des Prä-Proteins herrühren. Wir haben die Signalsequenz nicht entfernt, bevor wir das Antigen in großen Mengen herstellten, weil die Anwesenheit von Lipoylanteilen am N-terminalen Cystein eine wichtige Rolle in der Immunogenität von Proteinantigenen spielen könnte (6). Das rekombinante Protein, welches in dieser Erfindung gereinigt wurde, zeigte ebenfalls eine Doppelbandencharakteristik. Die Präparation I enthält hauptsächlich die obere Bande und nur geringe Mengen der unteren Bande. Präparation II enthält sowohl die obere als auch die untere Bande in fast gleichen Mengen. Präparation III repräsentiert ein proteolytisch verkürztes Derivat (etwa 33 kDa) des etwa 38 kDa großen Antigens.

Das native 38 kDa-Protein, welches aus dem Kulturüberstand von M. *tuberculosis* isoliert wird, und das gereinigte rekombinante Protein, das man in den Präparationen I und II findet, wiesen die gleiche Größe auf und zeigten auf SDS-PAGE-Gelen einen Doppelbandencharakter. Außerdem zeigten die gereinigten Proteine und das native Antigen auf Immunoblots eine identische Reaktion mit den monoklonalen Antikörpeern HAT2, HBT12 und HYT28. Ein polyklonales Serum, welches gegen das rekombinante Protein (Präparation I) gebildet wurde, erkannte das native Antigen und zeigte im ELISA die gleiche Reaktion wie ein Serum, das gegen das native Antigen gebildet wurde. Ähnliche Resultate erhielt man, wenn die beiden Seren im ELISA gegen das rekombinante Antigen getestet wurden, was zeigt, daß das rekombinante Protein ähnliche Epitope besitzt und so immunogen ist wie das native 38 kDa-Protein, das aus *M. tuberculosis* gereinigt werden kann.

Zusammenfassend haben wir ein Expressionssystem und ein Herstellungs- und Reinigungsverfahren für ein etwa 38 kDa großes Protein in E. coli auf Basis des 38 kDa-Proteins aus *M. tuberculosis* entwickelt, das eine leichte Isolation signifikanter Mengen des Antigens ermöglicht. Das rekombinante Antigen ist immunologisch nicht unterscheidbar von dem nativen Antigen.
Diese Ergebnisse sollten die Abschätzung der Wertigkeit des Antigens für Diagnostik und Entwicklung von Impfstoffen in signifikanter Weise beschleunigen.

### Literaturverzeichnis

1. Andersen, A.B., and E.B. Hansen. 1989.
   Structure and mapping of antigenic domains of protein antigen b, a 38.000-molecular-weight protein of *Mycobacterium tuberculosis.*
   Infect. Immun. 57:2481-2488.
2. Andersen, A.B., Z.-L. Yuan, K. Haslov, B, Vergmann, and J. Bennedsen. 1986. Interspecies reactivity of five monoclonal antibodies to *Mycobacterium tuberculosisas* as examined by immunoblotting and enzyme-linked immunosorbent assay.
   J. Clin. Microbiol. 23:446-451.
3. Andersen, A.B., A. Worsaae, and S.D. Chaparas. 1988.
   Isolation and characterisation of recombinant lambda gtll bacteriophages expressing eight different mycobacterial antigens of potential immunlological relevance.
   Infect. Immun. 56:1344-1351.
4. Bothamley, G.H., J.S. Beck, G.M.T. Schreuder, J. D'Amaro, R.R.P. de Vries, T. Kardijito, and J. Ivanyi. 1989.
   Association of tuberculosis and *Mycobacterium tuberculosis*-specific antibody level with HLA.
   J. Infect. Dis. 159:549-555.
5. Damerval, C., M. le Guilloux, J. Blaissonneau, and D. de Vienne. 1987. A simplification of Heukeshoven and Bernick's silver staining of proteins.
   Elektrophoresis 8:158-169.
6. Deres, K., H. Schild, K.-H. Wiesmüller, G. Jung, and H.G. Ramensee. 1989. *In vivo* priming of virus-specific cytotoxic T lymphocytes with synthetic lipopeptide vaccine.
   Nature 342:561-564
   Halenbeck, R., E. Kawasaki, J. Wrin, and K. Koths. 1989. Renaturation and purification of biologically active recombinant human macrophage colony-stimulating factor expressed in *Escherichia coli.*
   Biotechnology 7:710-715.
8. Hanahan, D. 1983. Studies on transformation of *Escherichia coli* with plasmids.
   J. Mol. Biol. 166:557-580.
9. Harboe, N., and A. Inglid. 1983. Immunization, isolation of immunoglobulins and antibody titre determination.
   Scand. J. Immunol. 17S10:345-351.
10. Kadival, G.V., S.D. Chaparas, and D. Hussong. 1987. Characterisation of serologic and cell-mediated reactivity of a 38 kDa antigen isolated from *Mycobacterium tuberculosis.*
   J. Immunol. 139:2447-2451.
11. Laemmli, U.K. 1970. Cleavage of structural proteins during the assembly of the head of bacteriophage T4.
   Nature (London) 227:680-683.
12. Ljungqvist, L., A. Worsaae, and I. Heron. 1988. Antibody resposes against *Mycobacterium tuberculosis* in 11 strains of inbred mice: novel monoclonal antibody specifities generated by fusions, using spleens from BALB.B10 and CBA/J mice.
   Infect. Immun. 56:1994-1998.
13. Lowry. O.H., A.L. Farr, N.J. Rosenbrough, and R. Randall. 1951. Protein measurement with the folin phenol reagent.
   J. Biol. Chem. 193:265-275.
14. McCarthy, J.E.G., and C. Bokelmann. 1988. Determinants of translational initiation efficiency in the *atp* operon of *Escherichia coli.*
   Molec. Microbiol. 2:455-465.
15. Maniatis, T., E.F. Fritsch, and J. Sambrook. 1982. Molecular cloning : a laboratory manual. Cold Spring Harbor Laboratory, Cold Spring Harbor , N.Y.
16. Pugsley, A.P., C. Chapon, and M. Schwartz. 1986. Extra-cellular pullulanase of *Klebsiella pneumoniae is* a lipoprotein.
   J. Bacteriol. 166:1083-1088.
17. Roth, J., M. Bendayan, E. Carlemalm, W. Villiger, and M. Garavito. 1981. Enhancement of structural preservation and immunocytochemical staining in low temperature embedded pancreatic tissue.
   J. Histochem. Cytochem. 29:663-669.
18. Sanger, F., S. Nicklen, and A.R. Coulson. 1977. DNA sequencing with chain-terminating inhibitors.
   Proc. Natl. Acad. Sci. USA 74:5463-5467.
19. Sarmientos, P., M. Duchesne, P. Denefle; J. Boiziau, N, Fromage, N. Delaporte, F. Parker, Y. Lelievre, J-F. Mayaux, and T. Cartwright. 1989. Synthesis and purification of active human tissue plasminogen activator from *Escherichia coli.*
   Biotechnology 7:495-501
20. Schauder, B., H. Blöcker, R. Frank, and J.E.G. McCarthy. 1987. Inducible expression vectors incorporating the *Escherichia coli atp*E translational initiation region.
   Gene. 52:279-283.
21. Schou, C., Z.-L. Yuan, A.B. Andersen, and J. Bennedsen. 1985. Production and partial characterisation of monoclonal hybridoma antibodies to *Mycobacterium tuberculosis.*
   Acta Pathol. Microbiol. Immunol. Scand. Sect. C 93:265-272
22. Schouls, L.M., R. Mount, J. Dekkert, and J.D.A. van Embden. 1989. Characterisation of lipid-modified immunogenic proteins of *Treponema palidum* expressed in *Escherichia coli.*
   Microbiol. Pathogen. 7:175-188.
23. Styblo, K. 1989. Overview and epidemiological assessment of the current global tuberculosis situation with an emphasis on control in developing countries.
   Rev. Infect. Dis. 11:5339-5346.
24. Worsaae, A., L. Ljungqvist, K. Haslov, I. Heron, and J. Bennedsen. 1987. Allergenic and blastogenic reactivity of three antigens from *Mycobacterium tuberculosis* in sensitized guinea pigs.
   Infect. Immun. 55:2922-2927.
25. Worsaae, A., L. Ljungqvist, and I. Heron. 1988. Monoclonal antibodies produced in BALB.B10 mice define new antigenic determinants in culture filtrate preparations of *Mycobacterium tuberculosis.*
   Infect. Immun. 56:2608-2614.
26. Young, D., L. Kent, A. Rees, J. Lamb, and J. Ivanyi. 1986. Immunological activity of a 38 kDa-kilodalton protein purified from *Mycobacterium tuberculosis.*
   Infect. Immun. 54:177-183.
27. Young, R.A., B.R. Bloom, C.M. Grosskinsky, J. Ivanyi, D.D. Thomas, and R.W. Davis. 1985. Dissection of *Mycobacterium tuberculosis* antigens using recombinant DNA.
   Proc. Natl. Acad. Sci. USA 82:2583-2587.
28. Zucker, M., and P. Stiegler. 1981. Optimal computer folding of large RNA sequences using thermodynamics and auxillary information.
   Nucl. Acids Res. 9:133-148.

### Lebendmaterial

- Lambda-AA59:: genomischer Klon für 2,0-kb-EcoRI-Fragment
Zugang: Literatur (3); DSM 6524
- M13mp19:: Phagen zur Transferierung für 2,0-kb-EcoRI-Fragment
Zugang: Pharmacia
- pJLA603:: Vektor zur Aufnahme eines NdeI-SphI-Fragments von M13mp19 nach Transferierung des 2,0-kb-EcoRI-Fragments und Mutagenisierung
Zugang: Literatur (20); Medac (Hamburg)

**Tabelle 1**

| Aminosäurenzusammensetzung des gereinigten 38 kDa-Antigens | | |
|---|---|---|
| Aminosäure | Anzahl der Reste | |
| | abgeleitet aus der DNA-Sequenz | Aminosäurenanalyse |
| Ala | 55 | 56.8 |
| | | |
| Arg | 5 | 6.9 |
| | | |
| Asn | 18 | 39.3 |
| Asp | 16 | |
| | | |
| Cys | 3 | ND^{a} |
| | | |
| Gln | 19 | 32.8 |
| Glu | 10 | |
| | | |
| Gly | 43 | 41.2 |
| | | |
| His | 7 | 7.6 |
| | | |
| Ile | 19 | 17.8 |
| | | |
| Leu | 36 | 32.4 |
| | | |
| Lys | 12 | 12.5 |
| | | |
| Met | 6 | 0.7 |
| | | |
| Phe | 13 | 13.0 |
| | | |
| Pro | 26 | 23.4 |
| | | |
| Ser | 26 | 22.3 |
| | | |
| Thr | 27 | 23.8 |
| | | |
| Trp | 4 | ND |
| | | |
| Tyr | 10 | 10.8 |
| | | |
| Val | 19 | 19.0 |

| | | |
|---|---|---|
| ^{a}ND nicht bestimmt | | |

## Patentansprüche

1. Hybrid-Plasmid zur Expression eines unfusionierten eine Prä-Sequenz aufweisenden etwa 38 kDa großen Antigens (Prä-Protein) von M. *tuberculosis* in *E. coli,* wobei das Plasmid
- eine DNA-Sequenz enthält, die 22 bis 17 Aminosäuren der folgenden Aminosäuresequenz kodiert: 
1                     23
MKIRLHTLLAVLTAAPLLLAAAG,
 und
- eine Restriktionsschnittstelle umfaßt, welche innerhalb ihrer Erkennungssequenz das Basentriplett ATG umfaßt, das die in Leserichtung erste Aminosäure M (Methionin) der in Fig. 1A dargestellten Wildtyp-Präsequenz des etwa 38 kDa großen Antigens kodiert.

2. Hybrid-Plasmid nach Anspruch 1, dadurch **gekennzeichnet,** daß es sich bei dem etwa 38 kDa großen Antigen um ein Protein einer *M*. -*tuberculosis-Variante* handelt, die ebenfalls Tuberkulose hervorrufen kann.

3. Hybrid-Plasmid nach einem der vorhergehenden Anprüche, dadurch **gekennzeichnet,** daß die das etwa 38 kDa große Antigen kodierende DNA-Sequenz mit ihrem 5'-Terminus in eine NcoI-, NdeI- oder SphI-Schnittstelle des Ausgangsvektors des Hybridplasmids eingesetzt worden ist, beispielsweise pJLA603 (Fig. 2) als Ausgangsvektor.

4. Hybrid-Plasmid nach einem der vorhergehenden Ansprüche, **gekennzeichnet** durch eine DNA-Sequenz gemäß Fig. 1 C.

5. E. coli mit einem Hybrid-Plasmid gemäß einem der vorhergehenden Ansprüche.

6. Etwa 38 kDa großes Antigen von *M. tuberculosis,* **herstellbar** mit Hilfe von *E. coli* gemäß Anspruch 5 und folgendermaßen renaturierbar:
- Solubilisieren des als Einschlußkörper angefallenen Antigens und/oder Proteins in Guanidinium-HCl (gegebenenfalls in Gegenwart eines reduzierenden Agens), z. B. mit etwa 6 M Guanidinium-HCl und/oder in Gegenwart von Dithiothreit (DTT) und
- nachfolgendes Renaturieren mit Hilfe von Sephadex-Chromatographie, z. B. mit Hilfe von Sephadex G-25.

7. Etwa 33 kDa großes *M. tuberculosis -Protein,* herstellbar mit Hilfe von *E. coli* als Wirt des Expressions-Plasmids pJLA 603 (Fig. 2), wobei
- eine das etwa 38 kDa große Antigen von *M*. *tuberculosis* kodierende DNA-Sequenz mit ihrem 5'-Terminus in eine NcoI-Schnittstelle von pJLA 603 eingesetzt worden ist,
- diese eingesetzte DNA-Sequenz die Signalsequenz des Antigens umfaßt und
- die Erkennungssequenz der Schnittstelle das Basentriplett ATG umfaßt, das die in Leserichtung erste Aminosäure M (Methionin) des etwa 38 kDa großen Antigens kodiert, und
separierbar vom gleichfalls exprimierten etwa 38 kDa großen Antigen.

## Claims

1. Hybrid plasmid for the expression of an unfused antigen of approx. 38 kDa having a pre-sequence (pre-protein) of *M*. *tuberculosis* in *E*. *coli,* the plasmid
- containing a DNA sequence which codes 22 to 17 amino acids in the following amino acid sequence: 
1                                    23
MKIRLHTLLAVLTAAPLLLAAG
 and
- comprises a restriction cleavage site which comprises, within its recognition sequence, the base triplet ATG which codes the first amino acid M (methionine) in the direction of reading of the wild-type presequence of the antigen of approx. 38 kDa illustrated in Fig. 1A.

2. Hybrid plasmid according to claim 1 characterised in that the antigen of approx. 38 kDa is a protein of a *M*. *tuberculosis* variant which is also capable of causing tuberculosis.

3. Hybrid plasmid according to one of the preceding claims characterised in that the DNA sequence coding the antigen of approximately 38 kDa has been inserted by its 5' end into a *NcoI, NdeI* or *SphI* cleavage site of the start vector of the hybrid plasmid, e.g. pJLA603 (Fig. 2) as start vector.

4. Hybrid plasmid according to one of the preceding claims characterised by a DNA sequence according to Fig. 1C.

5. *E. coli* with a hybrid plasmid according to one of the preceding claims.

6. Antigen of approx. 38 kDa of *M. tuberculosis,* preparable by means of *E*. *coli* according to claim 5 and renaturable as follows:
- solubilising of the antigen and/or protein, obtained as inclusion body, in guanidine HCl (if necessary in the presence of a reducing agent), e.g. with approx. 6 M guanidine HCl and/or in the presence of dithiothreitol (DTT) and
- subsequent renaturation by means of Sephadex chromatography, e.g. by means of Sephadex G-25.

7. *M. tuberculosis* protein of approx. 33 kDa preparable by means of *E*. *coli* as host of the expression plasmid pJLA 603 (Fig. 2)
- a DNA sequence coding the antigen of approximately 38 kDa of *M. tuberculosis* having been inserted by its 5' end into a *NcoI* cleavage site of pJLA603,
- this DNA sequence used comprising the signal sequence of the antigen and
- the recognition sequence of the cleavage site comprising the base triplet ATG which codes the first amino acid M (methionine) in the direction of reading and
separable from the equally expressed antigen of approx. 38 kDa.

## Revendications

1. Plasmide hybride pour l'expression, dans *E. coli*, d'un antigène de 38 kDa (pré-protéine) comportant une pré-séquence de *M. tuberculosis* lequel plasmide contient
- une séquence d'ADN codant de 22 à 17 acides aminés de la séquence d'acides aminés suivante : MKIRLHTLLAVLTAAPLLLAAAG et
- un site de restriction contenant, dans le site de reconnaissance le triplet de bases ATG qui code, dans le sens de la lecture, le premier acide acide animé M (méthionine) de la préséquence de type sauvage de l'antigène d'environ 38 kDa représenté dans le Figure 1A.

2. Plasmide hybride selon la revendication 1, caractérisé en ce que l'antigène d'environ 38 kDa est une protéine d'une variante de *M. tuberculosis* pouvant également provoquer la tuberculose.

3. Plasmide hybride selon l'une des revendications précédentes caractérisé en ce que la séquence d'ADN codant l'antigène d'environ 38 kDa est insérée avec son extrémité 5' au niveau d'un site de restriction *Nco*I*, Nde*I ou *Sph*I du vecteur de départ du plasmide hybride, par exemple le vecteur pJLA603 (Fig. 2).

4. Plasmide hybride selon une des revendications précédentes, caractérisé par une séquence d'ADN selon la Figure 1C.

5. *E*. *coli* contenant un plasmide hybride selon une des revendications précédentes.

6. Antigène d'environ 38 kDa de M. *tuberculosis,* que l'on peut préparer à l'aide de *E*. *coli* selon la revendication 5 et que l'on peut renaturer de la manière suivante :
- solubilisation de antigène et/ou de la protéine produit(e) sous forme de corps d'inclusion, dans du guanidinium-HCl (éventuellement en présence d'un agent de réduction), par exemple dans du guanidinium-HCl (6M) et/ou en présence de dithiothreit (DTT), et
- renaturation par chromatographie sur colonne de Sephadex, par exemple sur Sephadex G-25.

7. Protéine de *M. turberculosis* d'environ 33 kDa que l'on peut préparer en utilisant E. *coli* comme hôte hébergeant le plasmide d'expression pJLA 603 (Fig. 2),
- une séquence d'ADN codant l'antigène d'environ 38 kDa de *M. tuberculosis* ayant insérée avec son extrémité 5' dans le plasmide pJLA 603 au niveau d'un site de restriction *Nco*I*,*
- cette séquence d'ADN insérée englobant la séquence signal de l'antigène, et
- la séquence de reconnaissance du site de restriction englobant le triplet de bases ATG qui code, dans le sens de lecture, le premier acide aminé M (méthionine) de l'antigène d'environ 38 kDa, et
que l'on peut séparer de l'antigène d'environ 38 kDa également exprimé.
